(19) Europäisches Patentamt
European Patent Office
Office européen des brevets

(11) **EP 3 072 076 B1**

(12) **EUROPEAN PATENT SPECIFICATION**

(45) Date of publication and mention
of the grant of the patent:
**11.07.2018 Bulletin 2018/28**

(21) Application number: **14828322.9**

(22) Date of filing: **19.11.2014**

(51) Int Cl.:
***G06F 19/22*** *(2011.01)*

(86) International application number:
**PCT/NL2014/050792**

(87) International publication number:
**WO 2015/076671 (28.05.2015 Gazette 2015/21)**

(54) **A METHOD OF GENERATING A REFERENCE INDEX DATA STRUCTURE AND METHOD FOR FINDING A POSITION OF A DATA PATTERN IN A REFERENCE DATA STRUCTURE**

VERFAHREN ZUR ERZEUGUNG EINER REFERENZINDEXDATENSTRUKTUR UND VERFAHREN ZUM AUFFINDEN DER POSITION EINER DATENSTRUKTUR IN EINEM REFERENZDATENSTRUKTUR

PROCÉDÉ DE GÉNÉRATION D'UNE STRUCTURE DE DONNÉES D'INDEX DE RÉFÉRENCE ET PROCÉDÉ POUR TROUVER UNE POSITION D'UN MODÈLE DE DONNÉES DANS UNE STRUCTURE DE DONNÉES DE RÉFÉRENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.11.2013 NL 2011817**

(43) Date of publication of application:
**28.09.2016 Bulletin 2016/39**

(73) Proprietor: **Norlin Genalice Limited
Belfast BT1 2DY (GB)**

(72) Inventor: **KARTEN, Johannes
NL-3847 LW Harderwijk (NL)**

(74) Representative: **de Hoog, Johannes Hendrik
Octrooibureau de Hoog
Gouverneurslaan 18 A
3905 HE Veenendaal (NL)**

(56) References cited:
**EP-A1- 2 759 952          WO-A1-2014/060305
WO-A2-2010/104608**

• **NING Z: "SSAHA: a fast search method for large DNA databases", GENOME RESEARCH, COLD SPRING HARBOR LABORATORY PRESS, WOODBURY, NY, US, vol. 11, no. 10, 1 October 2001 (2001-10-01), pages 1725-1729, XP002983796, ISSN: 1088-9051, DOI: 10.1101/GR.194201**

**Description**

TECHNICAL FIELD

The invention relates to the field of alignment processes, and in particular, to a method of generating a reference index data structure enabling to find associated positions of M elements of a data pattern in a reference data structure and a method for finding associated positions of M elements of a data pattern in a reference data structure which is derived from one or more reference data files. More particularly, the invention relates to the process of mapping random reads from a sequence on a reference genome.

BACKGROUND

A genome exists of 4 different nucleotides or bases which form a string. The codes for these nucleotides are A C G T. These strings are connected where AT and CG form pairs. The bases can be encoded using two bits: 00-A, 01-C, 10-G, 11-T. The human genome exists of 3.2Billion base pairs. With an encoding of 2 bits per genome position, the entire genome can be stored in ~750Mb.

When alignment software looks for a pattern in the genome, the alignment software needs to compare the presented pattern with each possible pattern in the genome. When the software wants to 'match' a 50base pattern, i.e. a sequence or string of 50 bases, the software needs to compare this pattern with the pattern in de genome at position 1 then at 2, then at 3 at 4 etc. The genome is in the context of the present application regarded to be the reference data. A read, which is a sequence of M bases, is in the context of the present application regarded to be a data pattern for which the location on the reference data has to be found.

When the software finds a match, the software still has to look further, since there can be other locations in the reference data matching the pattern as well. Such pattern with more than one matching location in the reference data is called a repeat.

When the pattern does not match, the software must search for a partly match of the pattern in the reference data, so the software has to look again, but now with a fuzzy pattern to be able to locate the position.

Now a day several sequence alignment software tools are available on the market. Sequence alignment is the process of mapping RNA or DNA sequences on a reference DNA sequence. Such sequences could be in the form of a short Read of 75, 100, 150 or 200 bases. A high end sequencer can already produce 120Gbases per day in short reads. The number of reads which stream from a sequencer varies depending on the read size. The size of such a stream is in the order of 300Gbytes per day.

Current alignment tools can process 5-10 Gbases per day on a single CPU(core). Thus to process the sequences of short reads from a high end sequences in real time, about 20 CPU's have to work simultaneously. Thus if not enough CPU's are available, the amount of data generated by a high-end sequencer that is not processed will increase every day. Furthermore, due to the relative "slow" processing performance of the current alignment tools, the accuracy of the mapping is not 100% for algorithmic reasons which use a greedy approach. Furthermore, the reference sequence might change in time due to increased knowledge with a frequency of 1-2 years. This requires that the original input stream generated by a sequencer has to be mapped to or re-aligned with the new reference sequence. This requires additional computer systems with high capacity and high bandwidth networks to re-align the original input stream again.

Ning et al2001, (Genome: 11 :1725-1729), describes an algorithm, SSAHA (sequence search and alignment by hashing algorithm), for performing fast alignment on databases containing multiple gigabases of DNA. Sequences in the database are pre-processed by breaking them into consecutive k-tuples of k contiguous bases. A hash table is used to store the position of each occurrence of each k-tuple. Searching for a query sequence in the database is done by obtaining from the hash table the "hits" for each k-tuple in the query sequence and then performing a sort on the results. Storage of the hash table requires $4^{k+1}+8W$ bytes of RAM in all, in which k is the number of bases of a k-tuple and W is the number of k-tuples in the database.

WO2010/104608A2 describes a method for indexing a reference genome. The method calculates a first minimum index region size based on the reference genome size, for example on the fourth log of the total number of bases in the reference genome. Positions numbers are assigned to index regions with a size greater than or equal to the first minimum index region size. Each position number is a unique identifier for its respective sequence of nucleic acids. The association of the position numbers to index regions is stored in a hash table.

SUMMARY

It is an object of the invention to provide an improved method to reduce the processing time to find a match of a data pattern, e.g. a read of M bases, on a reference sequence. The method further removes the CPU bottleneck from the alignment process, allowing the process to run at the speed the bandwidth of memory, IO devices and inbound network connection permit. This is achieved by generating a comprehensive index with a memory footprint such that the index could be stored completely in the main memory of a CPU. The invention is defined by the claims. According to a first aspect of the invention, there is provided a computer-implemented method of generating a reference index data structure enabling to find associated positions of M elements of a data pattern in a reference data structure which is derived from one or more reference data files, the reference data structure comprises N reference elements and P padding elements. The padding elements follow the last reference element. Each element of the reference data structure has a position in the reference data structure and comprises an associated element value, and wherein N >> M. The method comprises the following actions:

- retrieving the reference data structure;
- generating from the reference data structure for the N references elements in the reference data structure an index structure which comprises for each of the N references an index entry which defines a data linkage between a position of an element in the reference data structure and a reference data pattern, the reference data pattern comprises K elements which are obtained by combining the element value at said position in the reference database and the element values of K-1 neighbouring positions of the element in the reference data structure, K being an integer smaller than P+1;
- sorting the index structure on the basis of the reference data patterns associated with each of the data linkages to obtain a sorted index, the sorted index comprises a data entry for each of the N reference elements of the reference data structure, a data entry comprises at least position information indicative for the position of a reference element in the reference data structure; and,
- storing the sorted index and the reference data structure in a data storage as reference index data structure.

The reference index data structure is subsequently used in computer-implemented method for finding associated positions of M elements of a data pattern in a reference data structure which is derived from one or more reference data files. The method comprises:

- retrieving the reference index data structure which comprises a sorted index and a reference data structure;
- searching through the sorted index to find the position of the M elements of the data pattern in the reference data structure; and,
- storing the data pattern and found position information for further processing. The searching action comprises:

    - reading position information from an entry of the sorted index;
    - generating a reference data pattern of M elements from the reference data structure using the position information;
    - comparing the data pattern with the reference data pattern; and,
    - stop searching when the entry in the index is found with the longest initial part of corresponding reference data pattern which matches a corresponding initial part of the data pattern or no matching initial part can be found, and,
    - providing the position of the entry with the longest initial part of corresponding reference data pattern which matches a corresponding initial part of the data pattern.

The principle of the present invention is that a new reference data index structure is generated prior to performing a search for a match. The reference data index structure enables to find quickly a perfect match of a read with variable length in the reference data structure and the number of locations the read perfectly matches the reference data structure. If there is only one location this means a unique match of the M bases of a read and if there are multiple locations the read could be marked a multi-reference hit. Furthermore, if no match could be found, the reference data index structure could be used to find efficiently the location(s) with the longest sequence that matches the beginning part of the M bases of the read to be matched. The new reference data index structure has to be generated once for each reference sequence or combination of reference sequences from one or more reference data files, for example retrieved from a FASTA file. Subsequently, the generated reference index data file only has to be read and no processing power is needed to prepare the index to make it suitable for matching with new short reads.

A reference index data structure which comprises an index structure in the form of a sorted index and a reference data structure has the advantage that the amount of memory needed to store the index structure could be reduced significantly. This can be explained as follows.

To locate the location of a pattern, i.e. string of bases, a complete index of the genome is build. Four bytes are needed to address the position of each base in the genome. The memory requirement for a complete index with a key-size of 64 bases (=128 bits = 16bytes) would be:

4bytes (position) + 16bytes (128 bits)/ position => 20 * 3.2Gbase = 64Gbytes.

This stores the position and the key in a linear list ordered on pattern, for example the binary representation of the patterns ordered from low to high. A key of 128 bits corresponds to a sequence of 64 bases in the reference data wherein the first base defines the location of the sequence in the reference data.

According to the present application, a sorted linked data structure is generated. The sorted linked data structure forms a dynamic Key Reference Index which does not store the key. Each entry of the sorted linked data structure only stores the 4byte position and uses this position to identify the location in the reference data structure. The key, which is referred to in the present application as reference data pattern, is subsequently reconstructed from the reference data structure. This reduces the full size of the reference index data structure to 3.2Gbase * 4 bytes = 12.8Gbytes for the sorted linked data structure and 750Mbytes for the reference data structure. This brings the total cost to 13.5 GB for the full genome index. The reference data structure is used as the key dictionary. It should be noted that in case a hash table, as described in "SSAHA: a fast search method for large DVA databases, would be used to store the position of each occurrence of the keys of 64 bases, the storage of the hash table would require 1,36E39 bytes.

A further advantage is that the size of this reference index data structure is independent of the size of the key as the key is no longer stored with the location identifier. It is only dependent on the size of the reference and the size of data required to address the location of the key. The organization of the sorted linked data structure forming the index structure can be a single ordered list, a tree structure, a hash structure or any other known index organization. The bigger the key-size, i.e. the number of elements or bases that has to be used to generate and order the index, the bigger the memory cost saving. Furthermore, the reference data index structure allows the index to be kept in modest size memory of a processor to speed up the pattern search for alignment.

The search through the index can be a binary search or an index tree traversal depending on the choice of the index organization structure.

The index does not only provide a quick path to a matching pattern, due to the ordering of the entries in the index structure, duplicates of varying size can be easily detected. The number of iterations which are required to find a match is depending on the size of the index, i.e. the number of entries which depends on the amount of reference elements in the reference data structure, and is about $\log 2$ of the number of positions in the reference data structure.

In the invention, a jump table with $2^J$ entries, is generated and used to find a match. J is an integer and corresponds to the number of bits representing an initial part of a reference data pattern. Each entry of the jump table has an implicit row number, a present field and an explicit jump field. The present field is used to indicate whether the sorted index comprises a reference data pattern which initial part is equal to the bits representing the row number. If the present field indicates that the sorted index comprises a data entry associated with a reference data pattern which initial part is equal to the bits representing the row number, a jump value is stored in the jump field which jump value refers to the first entry in the sorted index which corresponding reference data pattern has an initial part which matches the bits representing the row number of said entry of the jump table. The jump table is added to the reference index data structure and has to be generated once for a sorted index.

This mechanism reduces the number of iterations to find a match. The jump value of the entry which row number matches the beginning part of a data pattern defines the lower range of the search area in the sorted index and the jump value of the subsequent entry in the jump table which present field indicates that the sorted index comprises a reference data pattern which initial part is equal to the bits represented by its row number defines the upper range of the search area in the sorted index.

In an embodiment, a data entry of the sorted index further comprises a probe field which value corresponds to the element values of a part of the reference data pattern associated with the position information of said data entry. The probe field is used to be compared with the corresponding element values of a data pattern to be matched. Only if there is a perfect match, the sequence of K elements that forms the reference data pattern has to be generated from the reference data structure to compare the M elements of the data pattern with the corresponding K elements of a reference data pattern. In this way, the average processing time to verify whether a data pattern matches a reference data pattern associated with an entry of the index is reduced significantly.

For purposes outlined below, we do not only store the position (e.g. 4bytes) but also part of the key (2 bytes) and a multi-level frequency count (2 bytes). This totals the number of bytes for an index slot to 8. For a single full human genome index 8 * 3.2 G = 27 GB of memory would be required in this case.

According to a third aspect, there is provided a processing device comprising a processor, an Input/Output device to connect to the network system, a database and a data storage comprising instructions, which when executed by the processor cause the processing device to perform any of the methods described above.

Other features and advantages will become apparent from the following detailed description, taken in conjunction with the accompanying drawings which illustrate, by way of example, various features of embodiments.

## BRIEF DESCRIPTION OF THE DRAWINGS

These and other aspects, properties and advantages will be explained hereinafter based on the following description with reference to the drawings, wherein like reference numerals denote like or comparable parts, and in which:

Fig. 1 shows an example of a table with patterns and corresponding positions that could be deduced from a reference sequence to elucidate the principle of the present application;
Fig. 2 illustrates an example of the content of a sorted index derived from the reference sequence;
Fig. 3 illustrates an example of a jump table for the sorted index in Fig. 2;
Fig. 4 is a block diagram illustrating a computer implemented system.

## DETAILED DESCRIPTION

The following describes the techniques which are used to achieve near linear alignment performance. The technology removes the CPU bottleneck from the alignment process reaching the bandwidth of memory, IO devices and inbound network connections. Where current solutions achieve 5-10 Gbases/Day on a single CPU(core) this technology provides a throughput of 2500Gbase/Day utilizing 2.5 (2 GHz) cores.

Alignment is the process of mapping random reads on a reference data structure based on the pattern of the read. The number of reads which stream from a sequencer varies depending on the read size. A high end sequencer can produce 120Gbase per day in short reads of 75, 100, 150 or 200 bases. The size of such stream of bases generated by a sequencer is in the order of 300Gbytes.

The reference data structure could comprise the data of a genome. A genome exists of 4 different nucleotides or bases which form a string. The codes for these nucleotides are referred to in literature A C G T. These strings are connected where AT and CG form pairs. In the present application, the bases are encoded using two bits: 00 ≙ -A; 01 ≙ C; 10 ≙ G and 11 ≙ T. A human genome exists of 3.2Billion base pairs. With an encoding of 2 bits per genome position, the description of the entire genome can be stored in ~750Mb. The genome is a combination of a number of chromosomes of the DNA. In a FASTA file, which is commonly used to store a genome as reference data, each chromosome is described in a section of the FASTA file.

In the present application, a data pattern is a sequence of bases for example a read generated by a sequencer and a reference data structure is a data structure which includes the genome that is used as reference. When we want to know the matching location of a sequence of bases in the genome, we need to compare a data pattern with each possible location in the genome. When we find a match, we still have to look further, since there can be other positions matching the data pattern as well. Such a data pattern in the reference data structure is called in literature a repeat. When the pattern does not match there must be search for a partial match of the data pattern in the genome, so we have to look again, but now with a fuzzy pattern to be able to locate the position of the individual bases on the genome.

The concept of the present application is described in the present application by a very simplified example. The genome described by a sequence of 3.2Gbases is replaced with a reference data sequence describing two sections of 16 bases. In the following description of the method the term "element" refers a single base having a value A, C, G or T. The data pattern to be matched comprises instead of 50 or more elements only four (4) or more elements.

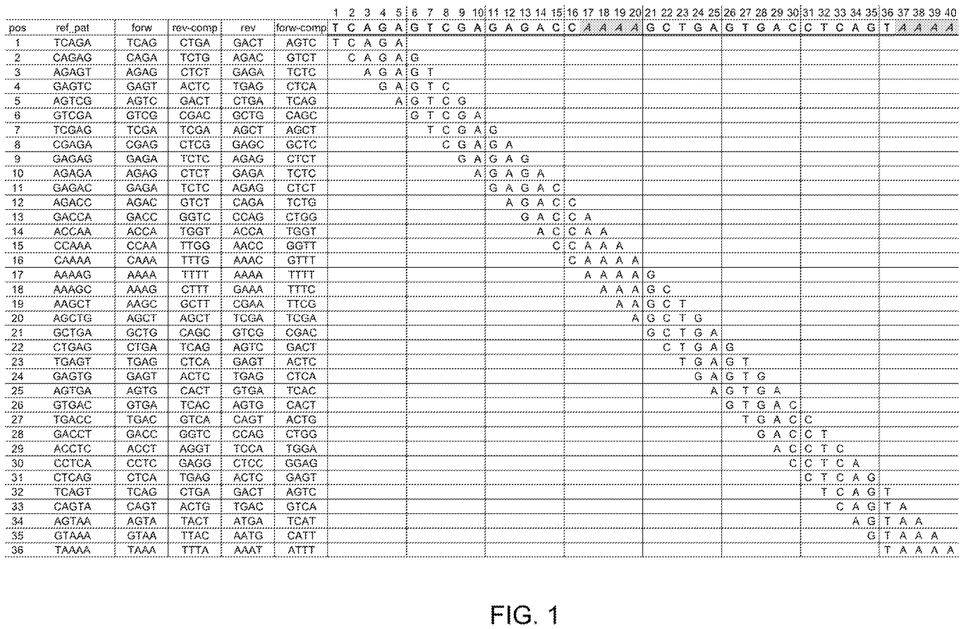

Fig. 1 shows table with on top the 40 positions of individual elements of the reference data structure and below each position the corresponding value of an element. The reference data structure describes two sections of a sequence of 16 elements. The elements of the first section are located at position 1 - 16 and the elements of the second section are located at position 21 - 36 in the reference data structure. Four padding elements are located at position 17 - 20 between the two sections and four padding element are positioned after the last section at position 37 - 40. In other words the four padding elements follow the last reference element.

As described before, to 'match' a base pattern with K elements, the sequence of K elements has to be compared with a reference data pattern of K elements at neighbouring positions in the reference data structure associated with any position of the elements of the reference data. In the present example we define K is 5. In the present example the reference data pattern at K neighbouring positions corresponds to K subsequent elements from the reference data structure wherein the first element of the sequence defines the position of the sequence in the reference data structure. To ensure that a matching reference data pattern from the reference data structure only comprises elements from one section, at least K - 1 elements have to be added between two sections. With K = 5 this means that at least 4, padding elements have to be added. Four padding elements added after the last section to be able to make a references data pattern for the last element of the reference data in the reference data structure. The sequence of the padding bits should be a sequence that has no significant meaning in the reference data. In the present example the sequence AAAA is used. It has been found advantageously to use the same padding sequence prior to the first section and following the last section in the reference data structure. For example, this allows using any of the K elements of the reference data pattern as anchoring point to define the position of the reference data pattern on the reference data structure.

The reference data structure could be obtained by reading a number of reference sequences or sections from one or more reference data files, for example FASTA files and generating the reference data structure by alternately adding a reference sequence and a predefined pattern of P padding elements to the reference data structure. P defines the maximum length K of reference data pattern that could be retrieved from the reference data structure wherein the reference data pattern comprises elements from only one reference sequence or section for which hold that K is an integer smaller than or equal to P+1.

In the table of Fig. 1, the first column indicated with "pos" comprises the position in the reference data structure. The second column indicated with "ref_pat" comprises the reference data patterns of 5 elements associated with each of the positions. The right part of the table illustrates how the reference data patterns are obtained. It can be seen that the reference data pattern at position 1 corresponds to the elements at position 1 - 5 in the reference data structure and that the reference data pattern for the last reference element of the last section at position 36 in the reference data structure corresponds to the elements at position 36 - 40. In this way, for each of the N references elements in the reference data structure a data linkage is created between a position in the reference data structure and a reference data pattern, the reference data pattern comprises K elements which are obtained by combining the element value at said position in the reference database and the element values of K-1 neighbouring positions of the element in the reference data structure. In this way a reference data pattern is associated with position in the reference data structure.

In practice for alignment of reads with 50, 100, 150 or 200 bases, the number N of elements in the reference data structure for a human genome is about $3.2 \times 10^9$ elements. A sequence of M elements for a data reference pattern, wherein M = 64 or 128 has been found suitable. It should be noted that with 64 elements, $4^{64} = 3 \times 10^{38}$ different sequences of 64 bases could be made. Thus the number of different reference data pattern of 64 bases from a reference genome compared with the theoretically possible combinations with 64 elements very low.

After generating a linkage between the relevant positions and the corresponding reference data structure, the data linkages are ordered on the basis of the reference data patterns associated with each of the data linkages to obtain a sorted index, the sorted index comprises a data entry for each of the N reference elements of the reference data structure. A data entry comprises at least position information indicative for the position of a reference element in the reference data structure. By means of the position, the data entry is associated with a reference data pattern.

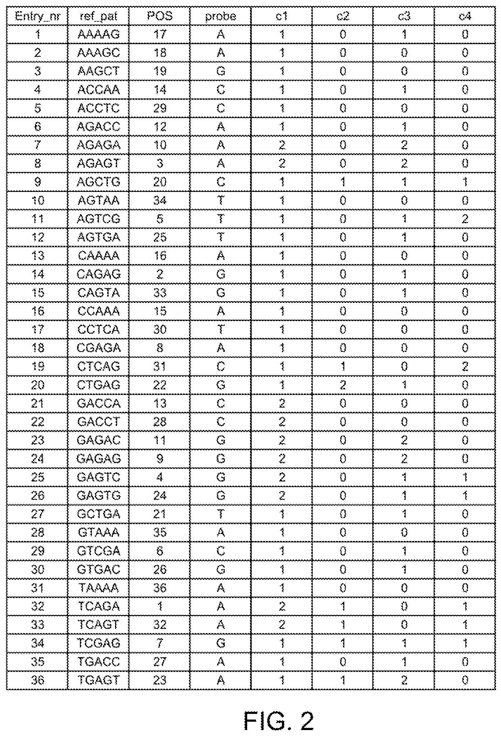

Fig. 2 illustrates an example of the content of a sorted index derived from the reference data structure of 40 elements shown in the table of figure 1. The first column indicated with "Entry_nr" comprises the entry numbers from 1 - 36. The third column indicated with POS comprises the starting position of a reference data pattern in the reference data structure. The starting position is stored in the corresponding data field of an entry. The sorted index forms an index to search for a particular data pattern with up to K elements, wherein K is defined by the number of elements of the reference data patterns that are used to sort the reference data patterns. It should be noted that the reference data pattern of K elements is not stored in a field of an entry. The value of K is a setting that can be chosen by a user.

The search through the index can be a binary search or an index tree traversal depending on the choice of the data structure of the sorted index. The size of this index is independent of the size of the key, i.e. the number of elements of a reference data pattern for sorting as the key is no longer stored with the location/position identifier. It is only dependent on the number of elements of the reference data structure and the size of data required to address the start position of the reference data pattern. The organization of the index structure can be a single ordered list, a tree structure a hash structure or any other known index organization. The bigger the sorting keySize, the bigger the memory cost saving. This organization structure allows the index to be kept in modest size memory to speed up the pattern search for alignment.

For example to find the location of data pattern "AGTGA" with a binary search through the sorted index, the algorithm start with reading the POS field of the entry in the middle of the 36 entries. Thus the value of the entry with number 18 will be read. The position value is 8. Subsequently, the element values at the positions 8 - 12 are read forming the reference data pattern "CGAGA". This value is higher than "AGTGA". If there is a matching value, the value has to be found in the entry range 1 - 17. Subsequently, the value of the pos field of the entry in the middle of the range 1 - 17 is read. The middle entry has the value 9 and the value of the pos field is 20. Subsequently, the element values at the positions 20 - 24 are read forming the reference data pattern "AGCTG". This value is lower than "AGTGA". If there is a matching value, the value has to be found in the entry range 10 - 17. Subsequently, the value of the pos field of the entry in the middle of the range 10 - 17 is read. The middle entry has the value 14 and the value of the pos field is 2. Subsequently, the element values at the positions 2 - 6 are read forming the reference data pattern "CAGAG". This value is higher than "AGTGA". If there is a matching value, the value has to be found in the entry range 10 - 13. Subsequently, the value of the pos field of the entry in the middle of the range 10 - 13 is read. The middle entry has the value 12 and the value of the pos field is 25. Subsequently, the element values at the positions 25 - 29 are read forming the reference data pattern "AGTGA". This value equals "AGTGA". Thus in four iterations the matching location is found.

The sorted index provides a quick path to a matching pattern, due to the ordering of the slots, duplicates of varying size can be easily detected. The maximum number of iterations which are required to find a match or no match depends on the size of the index, i.e. the number of elements of the reference data structure and is on average the log2 of the size.

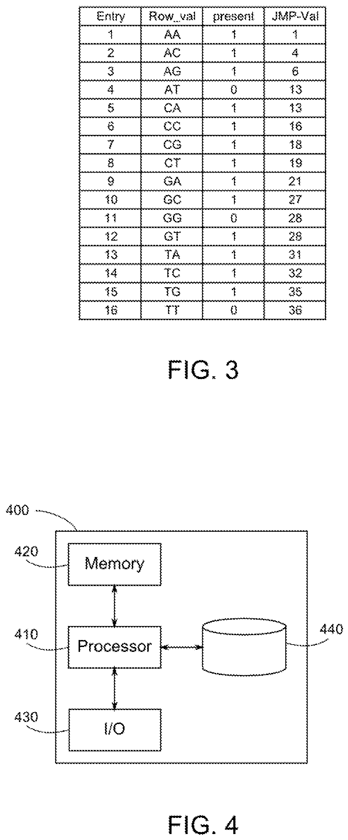

To speed up the search algorithm a jump table could be used as illustrated in Fig. 3. In the present example the first two elements of the data pattern to be matched are used to determine the entry number or the row value of the entry comprising the jump value to the sorted index. An entry of the jump table comprises a present field, the third column of the table in Fig. 3 and a jump field, the third column indicated with JMP-Val. The value of the present field indicates whether the row value Row_val is a data pattern that is present in the reference data structure. The value of the jump field of an entry in the jump table which row value corresponds to a data pattern in the reference data structure refers to the first entry in the sorted index which corresponding reference data pattern starts with a sequence of elements equal to the Row_val of said entry. If the value of the row value is a data pattern that does not exist in the reference data structure, the jump value JMP-Val might be any value. It is advantageous to assign the jump value of the next entry of the jump table which present field indicates that the Row_val represents a data pattern that exist in the reference data structure. In that case, when searching for a match for a data pattern, the jump value of the entry which row value Row_val matches the initial part of the data pattern defines the lower limit of the search area in the sorted index and the row value of the next entry defines the upper limit of the search area. In the table of Fig. 3, a "1" in the present field indicates that the data pattern represented by the row value is present and a "0" indicates that the data pattern represented by the row value is not present in the reference data structure. It can be seen for the jump table that the data patterns "AT", "GG" and "TT" does not exist in the reference data structure shown in Fig. 1.

Now for searching a match for data pattern "AGTGA", the jump table will provide the lower limit 6 and the upper limit 13. Subsequently, the value of the pos field of the entry in the middle of the range 6 - 12 is read. The middle entry has the value 9 and the value of the pos field is 20. Subsequently, the element values at the positions 20 - 24 are read forming the reference data pattern "AGCTG". This value is lower than "AGTGA". If there is a matching value, the value has to be found in the entry range 10 - 12. Subsequently, the value of the pos field of the entry in the middle of the range 10 - 12 is read. The middle entry has the value 11 and the value of the pos field is 5. Subsequently, the element values at the positions 5 - 9 are read forming the reference data pattern "AGTCG". This value is lower than "AGTGA". If there is a matching value, the value has to be found in the entry 12. The value of the pos field of entry 12 is 25. Subsequently, the element values at the positions 25 - 29 are read forming the reference data pattern "AGTGA". This value equals "AGTGA". Thus in three iterations the matching location is found.

On average, by using a jump table the number of iterations is decreased with the number of elements represented by the row value of an entry of the jump table. If a data pattern starts with a pattern that does not exist, the present field of the jump table will indicate that it is not necessary to access the sorted linkage table to find a position to reconstruct a reference data pattern from the reference data patterns. Thus without any access of the reference data structure it is known that no matching pattern can be found.

In Fig. 3 an entry of the jump table comprises a present field and a jump field. It might be clear that other implementations are possible. An example is using the sign-bit of the jump field as present indicator. In that case an entry of the jump table comprises only the jump field.

The number of times a reference data pattern has to be reconstructed from the reference data structure could be further reduced by adding a probe field to each entry of the sorted index. In the table shown in Fig. 2, the value of the probe fields are shown in the fourth column. The value of the probe field corresponds to the value of the third element of the reference data pattern.

Given the example with data pattern "AGTGA", the jump table will provide the lower limit 6 and the upper limit 13. Subsequently, the value of the pos field of the entry in the middle of the range 6 - 12 is read. The middle entry has the value 9 and the value of the corresponding probe field is "C". This value is lower than the third element of "AGTGA", namely T. If there is a matching value, the value has to be found in the entry range 10 - 12. Subsequently, the value of the pos field of the entry in the middle of the range 10 - 12 is read. The middle entry has the value 11 and the value of the corresponding probe field is "T". This value equals the third element of "AGTGA". Subsequently, the value of the pos field 5 is read, and the element values at the positions 5 - 9 in the reference data structure are read forming the reference data pattern "AGTCG". This value is lower than "AGTGA". If there is a matching value, the value could only be found in the entry 12. Now first the value of the probe field is compared with the value of the third element of the data pattern. As the value equals the third element, subsequently the value of the pos field of entry 12 is 25 used to reconstruct the reference data pattern from the reference data structure. The element values at the positions 25 - 29 form the reference data pattern "AGTGA". This value equals "AGTGA" and a perfect match is found. Thus again in three iterations the matching location is found however only twice the reference data pattern has to be reconstructed from the reference data structure.

As the index in the form of a sorted index has to be made once for a reference data structure and does not depend on the data patterns to be matched, additional information related to the reference data structure could be stored in the sorted index.

As described before, the sorted index in Fig. 2 is sorted based on reference data pattern comprising 5 element. The sorted index could also be used to search for a match in the reference data structure of a pattern with less than 5 elements. The possibility that a data pattern has more than one match in the reference data structure increases with reduction of the length of the data pattern. In alignment process it is important to know whether a data pattern has only one unique matching location in the reference data structure or multiple matches.

The entries of the sorted index could comprise a first count field indicating the number of times a reference data pattern with a predefined length has a matching location on the reference data pattern. In the table of Fig. 2 illustrating the content and associated reference data pattern of 5 elements associated with the position in the field "POS" additional count field $c_1$ ... $c_4$ are added to each entry. The value of count field $c_1$ corresponds to the number of times or frequency a data pattern corresponding to the first four elements of the corresponding reference data pattern exist in the reference data pattern. The length of the data pattern to determine the count values is a setting that may be adapted by a user. As the reference patterns are already sorted, it is very easy to find and count the data patterns having the same initial part of four elements. For example, the data pattern "AGAG" occurs two times in the reference data pattern and can be found in subsequent entries 7 and 8 of the sorted index. In the table of Fig. 1 the reference data pattern comprising four elements are shown in the third column indicate with "forw".

Read patterns which are produced by a sequencer do not have a defined 'constellation'. A read can be obtained from the so called plus-strand (one side of the DNA helix) or from the min-strand, i.e. the opposite side of the helix. To be able to find patterns which are read from either side aligners usually maintain two sets of indexes. One with the reference pattern in the forward direction and one with the reference pattern in the reverse complement direction. When for both indexes a full and complete index is build, this would require 128 GB of memory.

On top of the memory savings due to the index organisation, we utilize a single index to find all constellations. We search the index for matches for all reads which are presented to us in different constellations. We present the read as is, we do an inverse-complement of the read to project the pattern on the reverse strand and if required we can set the read in simple complement or in inverse mode. Thus a read can be forward or reverse and it can be normal or complement. This means that a produced read can be:

> ACGTAATT - forward
> TGCATTAA - forward-complement
> TTAATGCA - reverse
> AATTACGT - reverse-complement

Which in fact is exactly the same pattern. When a read is mapped on a reference data structure, all constellations (forward, forward-complement, reverse and reverse-complement) for a single read have to be checked to find all perfect matching locations for said read. This would mean that we first match the normal read (forward) and subsequently the other constellations. However, as the possible matching reference data patterns are known, the other constellations of said matching reference data patterns could be derived from the matching reference data patterns and subsequently the number of perfect matching location could be determined for each of the constellation.

As said before, in column 3 of the table of Fig. 1, the possible forward reference data patterns of four elements are shown. Columns 4, 5 and 6 of the table of Fig. 1 shows the corresponding data patterns of the reverse-complement constellation, the reverse constellation and the forward-complement constellation respectively. These data patterns are subsequently used to determine for each of the values the number of times the constellation occurs in the reference data structure. Columns 6, 7 and 8 indicated with $c_2$, $c_3$ and $c_4$ in Fig. 2 indicate the number of times a data pattern being a constellation of the first four elements of the reference data pattern corresponding to the position indicated in the POS field occurs in the reference data structure. For example entry number 11 corresponds to the reference data pattern "AGTCG" which starts at position POS 5 in the reference data structure. Field $c_1$ of entry number 11 comprises the value 1 which means that the data pattern "AGTC" occurs once in the reference data pattern. Field $c_3$ of entry number 11 comprises the value 1 which means that reverse of data pattern "AGTC", which is "CTGA" occurs once in the reference data pattern. Field $c_4$ of entry number 11 comprises the value 2 which means that complement of data pattern "AGTC", which is "TCAG" occurs twice in the reference data pattern, namely starting a position 1 and 32. The count values in the field $c_1$, $c_2$, $c_3$ and $c_4$ could be very helpful in determining a quality factor for a read in a further processing method which performed after the method described in the present application. As the count values only depend on the desired pattern length and the reference data structure, the can be determined once and used multiple times.

It might be clear that depending on the implementation, an entry of the sorted index could have any combination of the count fields. It might even be possible that the values of two or three count field are summated and stored in one field. The length of the data pattern to determine the count values might be set by a user and could have any value that is not higher than the value K which defines the length of the reference data patterns to generate the sorted index.

The above described reference data structure, sorted index and jump table form together a reference index data structure. The three components of the reference index data structure could be stored as one single file, three individual files or any other combination. If the index and reference data structure are stored as separate files, the index comprises a parameter identifying the corresponding reference data structure.

Described above is a method of generating a reference index data structure enabling to find associated positions of M elements of a data pattern in a reference data structure. The reference data structure comprises N reference elements and P padding elements. Each element has a position in the reference data structure and comprises an associated element value, and wherein N >> M. The method comprises retrieving the reference data structure, generating from the reference data structure for each of the N positions in the reference data structure a data linkage between a position of an element in the reference data structure and a reference data pattern. The reference data pattern comprises K elements which are obtained by combining the element value at said position in the reference database and the element values of K-1 neighbouring positions of the element in the reference data structure, K being an integer smaller than or equal to P+1. The data linkages are logically sorted on the basis of the reference data patterns associated with each of the data linkages to obtain a sorted index. The sorted index comprises a data entry for each of the N reference elements of the reference data structure. A data entry comprises at least position information indicative for the position of a reference element in the reference data structure. The sorted index and the reference data structure are stored in a data storage as a reference index data structure.

Furthermore, a jump table could be generated with $2^J$ entries, J is an integer and corresponds to the number of bits representing an initial part of a reference data pattern, each entry of the jump table having a row number, a present field and a jump field, the present field is used to indicate whether the sorted index comprises a reference data pattern which initial part is equal to the bits representing the row number. If the present field indicates that the sorted index comprises a reference data pattern which initial part is equal to the bits representing the row number a jump value is stored in the jump field which jump value refers to the first entry in the sorted index which corresponding reference data pattern has an initial part which matches the bits representing the row number of said entry of the jump table. The jump table is added to the reference index data structure.

Furthermore, a method for finding associated positions of M elements of a data pattern in a reference data structure which is derived from one or more reference data files is described. The method comprises: retrieving a reference data structure and a sorted index as described in the present application, an index structure which is derived from the reference data structure and optionally a jump table. The reference data structure, the sorted index and optionally the jump table are used to search for one or more matching locations for the M elements of the data pattern in the reference data structure. The data pattern and found position information and optionally particular count information are stored for further processing.

During a searching action, position information is read from an entry of the sorted linkage structure, a reference data pattern of M elements is generated from the reference data structure using the position information and the data pattern is compared with the reference data pattern. The searching action is stopped when the entry in the index is found with the longest initial part of corresponding reference data pattern which matches a corresponding initial part of the data pattern or no matching initial part can be found. The position of the entry with the longest initial part of corresponding reference data pattern which matches a corresponding initial part of the data pattern is stored for further processing in a subsequent alignment process.

The described methods are performed on a computer implemented system. As the described method for matching data patterns could process the stream of a sequencer in real time, it might be possible to integrate the alignment process which includes the process for finding associated positions of M elements of a data pattern in a reference data structure in a sequencer.

Referring to Fig. 4, there is illustrated a block diagram of exemplary components of a computer implemented system 400. The computer implemented system 400 can be any type of computer having sufficient memory and computing resources to perform the described method. As illustrated in Fig. 4, the processing unit 400 comprises a processor 410, data storage 420, an Input/Output unit 430 and a database 440. The data storage 420, which could be any suitable memory to store data, comprises instructions that, when executed by the processor 410 cause the computer implemented system 400 to perform the actions corresponding to any of the methods described in the present application. The data base could be used to store the reference index data structure, the data patterns to be matched and the results of the methods.

The method could be embodied as a computer program product comprising instructions that can be loaded by a computer arrangement, causing said computer arrangement to perform any of the methods described above. The computer program product could be stored on a computer readable medium.

A product of the method of generating a reference index data structure is a database product comprising a reference data structure, a sorted index and optionally a jump table.

Described is an index structure with entries in which we maintain the key/pattern frequency with each position where the associated data pattern is found. It allows the registration of repeat patterns from all mentioned constellations (forward/forward-complement/reverse/reverse-complement) with each position representing the anchor point of the pattern. When not checking for pattern frequency perfect matches on such repeat pattern will be mapped onto the first encountered match. When checking for frequency (total of count values of any constellation), mapping of the pattern can be deferred till after the read mate (in case of pairEnd mapping) such that pair-data can be used to properly map the read.

A short probe can be stored with the position to accelerate the index search. This functions as a modest key cache to facilitate better CPU cache utilization and thus performance. The binary search method through the index will first decide direction on the key prefix and will construct the reference data pattern (which is much bigger e.g. 128base versus 8base) on probe equality only.

Next to alignment it is clear that the above index method can be generally used for a variety of pattern matching purposes which consist of a reasonably static part (the reference) and a more dynamic part (the test set). The described methods could also be used to map fragments of an image on a reference image.

**Claims**

1. A computer-implemented method of generating a reference index data structure enabling to find associated positions of M elements of a data pattern in a reference data structure, the reference data structure comprises N reference elements and P padding elements following the last reference element, each element having a position in the reference data structure and comprises an associated element value, and wherein N >> M, the method comprising:

   - retrieving the reference data structure;
   - generating from the reference data structure for each of the N references elements in the reference data structure an index structure which comprises for each of the N reference elements an index entry which defines a data linkage between a position of an element in the reference data structure and a reference data pattern, the reference data pattern comprises K elements which are obtained by combining the element value at said position in the reference database and the element values of K-1 neighbouring positions of the element in the reference data structure, K being an integer smaller than or equal to P+1;
   - sorting the index structure on the basis of the reference data patterns associated with each of the data linkages to obtain a sorted index, the sorted index comprises a data entry for each of the N reference elements of the reference data structure, a data entry comprises at least position information indicative for the position of a reference element in the reference data structure; and,
   - storing the sorted index and the reference data structure in a data storage as reference index data structure,

   **characterized in that**, the method further comprises:

   - generating a jump table with $2^J$ entries, J is an integer and corresponds to the number of bits representing an initial part of a reference data pattern, each entry of the jump table having a row number, a present field and a jump field, the present field is used to indicate whether the sorted index comprises a data entry associated with a reference data pattern which initial part is equal to the bits representing the row number, if the present field indicates that the sorted index comprises a data entry associated with a reference data pattern which initial part is equal to the bits representing the row number a jump value is stored in the jump field which jump value refers to the first entry in the sorted index which corresponding reference data pattern has an initial part which matches the bits representing the row number of said entry of the jump table, and
   - adding the jump table to the reference index data structure.

2. The method according to claim 1, wherein a data entry of the sorted index further comprises a probe field which value corresponds to the element values of a part of the reference data pattern associated with the position information of said data entry.

3. The method according to claim 2, wherein the value of the probe field corresponds to the element values of a part of the reference data pattern which is subsequent to the initial part of the data pattern represented by a row number of the jump table.

4. The method according to any of the claims 1 - 3, wherein a data entry of the sorted index further comprises one or more count fields, the method further comprises:

   - determining for each entry in the sorted index the number of entries in the sorted index whose associated data pattern is identical to the data pattern associated with said entry;
   - determining for each entry in the sorted index the number of entries in the sorted index whose associated data pattern is identical to the data pattern associated with said entry in reverse complement order;
   - determining for each entry in the sorted index the number of entries in the sorted index whose associated data pattern is identical to the complement and/or reverse data pattern of the data pattern associated with said entry; and,
- storing one or more of the determined numbers and/or a summation of one or more of the determined numbers in the one or more count fields.

5. The method according to any of the claims 1 - 4, wherein the retrieving the reference data structure action comprises:

- reading a number of reference sequences from one or more reference data files;
- generating the reference data structure by alternately adding a reference sequence and a predefined pattern of P padding elements to the reference data structure.

6. The method according to any of the claims 1 - 5, wherein the reference index data structure is in the form of a data file.

7. A computer-implemented method for finding associated positions of M elements of a data pattern in a reference data structure which is derived from one or more reference data files; the method comprises:

- retrieving an index structure which is derived from the reference data structure;
- searching through the index structure to find the position of the M elements of the data pattern in the reference data structure; and,
- storing the data pattern and found position information for further processing, the reference data structure comprises N reference elements and P padding elements following the last reference element, each element having a position in the reference data structure and comprises an associated element value, and wherein $N \gg M$,

the index structure is a sorted index that has been generated by:

- generating from the reference data structure for each of the N references elements in the reference data structure an index structure which comprises for each of the N reference elements an index entry which defines a data linkage between the position of the element and a reference data pattern comprising K elements, a reference data pattern is obtained by combining the element value at said position in the reference database and the element values of K-1 neighbouring positions of the element in the reference data structure, K being an integer smaller than or equal to P+1;
- sorting the index structure on the basis of the reference data patterns associated with each of the data linkages to obtain a sorted index, the sorted index comprises a data entry for each of the N reference elements of the reference data structure, a data entry comprises at least position information indicative for the position of a reference element in the reference data structure; and,

the searching action comprises:

- reading position information from an entry of the sorted linkage structure;
- generating a reference data pattern of M elements from the reference data structure using the position information;
- comparing the data pattern with the reference data pattern; and
- stop searching when the entry in the index is found with the longest initial part of corresponding reference data pattern which matches a corresponding initial part of the data pattern or no matching initial part can be found, and
- providing the position of the entry with the longest initial part of corresponding reference data pattern which matches a corresponding initial part of the data pattern, **characterised in that**, the method further comprises:

- retrieving a jump table with $2^J$ entries, J is an integer and corresponds to the number of bits representing an initial part of a reference data pattern, each entry of the jump table having a row number, a present field and a jump field, the present field is used to indicate whether the sorted index comprises a data entry associated with a reference data pattern which initial part is equal to the bits representing the row number, if the present field indicates that the sorted index comprises a data entry associated with a reference data pattern which initial part is equal to the bits representing the row number a jump value is stored in the jump field which jump value refers to the first entry in the sorted index which corresponding reference data pattern has an initial part with predetermined size which matches the bits representing the row number of said entry of the jump table, and the searching action further comprises:

- retrieving an initial part with the predetermined size from the data pattern;

- retrieving a first jump value from the entry of the jump table which bits representing row number corresponds to the initial part of the data pattern and a second jump value corresponding the next entry of the jump value which present field indicates that the sorted index comprises a data entry associated with a reference data pattern which initial part is equivalent to the bits representing the row number;
- using the first and second jump value to define a search area in the sorted index.

8.  The method according to claim 7, wherein a data entry of the sorted index further comprises a probe field which value corresponds to the element values of a part of the reference data pattern associated with the position information of said data entry; the searching action further comprises reading the value of the probe field from the entry of the sorted index; and performs the generating action if a predefined part of the data pattern matches the value of the probe field.

9.  The method according to claim 8, wherein the value of the probe field corresponds to the element values of a part of the reference data pattern which is subsequent to the initial part of the data pattern which is represented by a row number of the jump table.

10.  The method according to any of the claims 7 - 9, wherein a data entry of the sorted index further comprises one or more count fields, a value of a count field represent the number of entries in the sorted index whose associated data pattern of K elements is identical to at least one of: the data pattern associated with said entry, to the data pattern associated with said entry in reverse complement order, the complement data pattern associated with said entry, reverse data pattern of the data pattern associated with said entry; and wherein the method further comprises:

- reading the one or more count fields from the entry which associated reference data pattern matches the data pattern and storing a corresponding link between a representation of the data pattern and the corresponding value of the one or more count fields in a data storage for further processing.

11.  The method according to any of the claims 7 - 10, wherein the reference data structure and index structure are retrieved from a data storage in which a reference index data structure obtained by the method according to any of the claims 1 - 6 is recorded.

12.  A computer implemented system (400) comprising a processor (410), an Input/Output device (430), a database (440) and a data storage (420) connected to the processor, the data storage comprising instructions, which when executed by the processor (410), cause the computer implemented system to perform the methods according to any of the claims 1 - 11.

13.  A computer program product comprising instructions that can be loaded by a computer arrangement, causing said computer arrangement to perform any of the methods according to claims 1 - 11.

14.  A processor readable medium provided with a computer program product comprising instructions that can be loaded by a computer arrangement, causing said computer arrangement to perform any of the methods to claims 1 - 11.

15.  A database product comprising a reference index data structure generated by any of the methods according to claims 1 - 6.

**Patentansprüche**

1.  Computerimplementiertes Verfahren zur Erzeugung einer Referenzindexdatenstruktur, die das Auffinden zugeordneter Positionen von M Elementen eines Datenmusters in einer Referenzdatenstruktur ermöglicht, wobei die Referenzdatenstruktur N Referenzelemente und P Füllelemente umfasst, die dem letzten Referenzelement folgen, wobei jedes Element eine Position in der Referenzdatenstruktur aufweist und einen zugeordneten Elementwert umfasst, und wobei N » M ist, das Verfahren umfassend:

- Abrufen der Referenzdatenstruktur;
- Erzeugen, aus der Referenzdatenstruktur, für jedes der N Referenzelemente in der Referenzdatenstruktur, einer Indexstruktur, die für jedes der N Referenzelemente einen Indexeintrag umfasst, der eine Datenverknüpfung zwischen einer Position eines Elements in der Referenzdatenstruktur und eines Referenzdatenmusters definiert, wobei das Referenzdatenmuster K Elemente umfasst, die durch Kombinieren des Elementwerts an der Position in der Referenzdatenbank und den Elementwerten von K-1 benachbarten Positionen des Elements in der Referenzdatenstruktur erhalten werden, wobei K ein ganze Zahl kleiner als oder gleich P+1 ist;
- Sortieren der Indexstruktur basierend auf den Referenzdatenmustern, die jeder der Datenverknüpfungen zugeordnet sind, um einen sortierten Index zu erhalten, wobei der sortierte Index einen Dateneintrag für jedes der N Referenzelemente der Referenzdatenstruktur umfasst, wobei ein Dateneintrag mindestens eine Positionsinformation umfasst, welche die Position eines Referenzelements in der Referenzdatenstruktur angibt; und
- Speichern des sortierten Index und der Referenzdatenstruktur in einem Datenspeicher als Referenzindexdatenstruktur,

**dadurch gekennzeichnet, dass** das Verfahren ferner umfasst:

- Erzeugen einer Sprungtabelle mit $2^J$ Einträgen, wobei J eine ganze Zahl ist und der Anzahl von Bits entspricht, die einen Anfangsabschnitt eines Referenzdatenmusters darstellen, wobei jeder Eintrag in der Sprungtabelle eine Zeilennummer, ein vorliegendes Feld und ein Sprungfeld aufweist, wobei das vorliegende Feld verwendet wird, um anzugeben, ob der sortierte Index einen Dateneintrag umfasst, der einem Referenzdatenmuster zugeordnet ist, dessen Anfangsabschnitt gleich den Bits ist, welche die Zeilennummer darstellen, wobei, wenn das vorliegende Feld angibt, dass der sortierte Index einen Dateneintrag umfasst, der einem Referenzdatenmuster zugeordnet ist, dessen Anfangsabschnitt gleich den Bits ist, welche die Zeilennummer darstellen, ein Sprungwert in dem Sprungfeld gespeichert wird, dessen Sprungwert sich auf den ersten Eintrag in dem sortierten Index bezieht, dessen jeweiliges Referenzdatenmuster einen Anfangsabschnitt aufweist, der mit den Bits übereinstimmt, die die Zeilennummer des Eintrags in der Sprungtabelle darstellen, und
- Hinzufügen der Sprungtabelle zu der Referenzindexdatenstruktur.

2. Verfahren nach Anspruch 1, wobei ein Dateneintrag des sortierten Index ferner ein Prüffeld umfasst, dessen Wert den Elementwerten eines Abschnitts des Referenzdatenmusters entspricht, welcher der Positionsinformation des Dateneintrags zugeordnet ist.

3. Verfahren nach Anspruch 2, wobei der Wert des Prüffelds den Elementwerten eines Abschnitts des Referenzdatenmusters entspricht, welcher dem Anfangsabschnitt des Datenmusters folgt, das durch eine Zeilennummer der Sprungtabelle dargestellt wird.

4. Verfahren nach einem der Ansprüche 1 - 3, wobei ein Dateneintrag des sortierten Index ferner ein oder mehrere Zählfelder umfasst, wobei das Verfahren ferner umfasst:

- Bestimmen, für jeden Eintrag in dem sortierten Index, der Anzahl von Einträgen in dem sortierten Index, deren zugeordnete Datenmuster mit dem Datenmuster identisch sind, das dem Eintrag zugeordnet ist;
- Bestimmen, für jeden Eintrag in dem sortierten Index, der Anzahl von Einträgen in dem sortierten Index, deren zugeordnete Datenmuster mit dem Datenmuster identisch sind, das dem Eintrag in umgekehrter komplementärer Reihenfolge zugeordnet ist;
- Bestimmen, für jeden Eintrag in dem sortierten Index, der Anzahl von Einträgen in dem sortierten Index, deren zugeordnete Datenmuster mit dem komplementären und/oder umgekehrten Datenmuster des Datenmusters identisch sind, das dem Eintrag zugeordnet ist; und
- Speichern einer oder mehrerer der bestimmten Zählwerte und/oder einer Summierung eines oder mehrerer der ermittelten Zählwerte in dem einen oder den mehreren Zählfeldern.

5. Verfahren nach einem der Ansprüche 1 - 4, wobei das Abrufen der Referenzdatenstrukturaktion umfasst:

- Auslesen einer Anzahl von Referenzsequenzen aus einer oder mehreren Referenzdatendateien;
- Erzeugen der Referenzdatenstruktur durch abwechselndes Hinzufügen einer Referenzsequenz und eines vordefinierten Musters von P Füllelementen zu der Referenzdatenstruktur.

6. Verfahren nach einem der Ansprüche 1 - 5, wobei die Referenzindexdatenstruktur die Form einer Datendatei aufweist.

7. Computerimplementiertes Verfahren zum Auffinden von zugeordneten Positionen von M Elementen eines Datenmusters in einer Referenzdatenstruktur, die von einer oder mehreren Referenzdatendateien abgeleitet wird; wobei das Verfahren umfasst:

- Abrufen einer Indexstruktur, die von der Referenzdatenstruktur abgeleitet wird;
- Durchsuchen der Indexstruktur, um die Position der M Elemente des Datenmusters in der Referenzdatenstruktur zu finden; und
- Speichern des Datenmusters und der gefundenen Positionsinformation zur weiteren Verarbeitung, wobei die Referenzdatenstruktur N Referenzelemente und P Füllelemente umfasst, die dem letzten Referenzelement folgen, wobei jedes Element eine Position in der Referenzdatenstruktur aufweist und einen zugeordneten Elementwert umfasst, und wobei N » M ist,

wobei die Indexstruktur ein sortierter Index ist, der durch Folgendes erzeugt wurde:

- Erzeugen, aus der Referenzdatenstruktur, für jedes der N Referenzelemente in der Referenzdatenstruktur, einer Indexstruktur, die für jedes der N Referenzelemente einen Indexeintrag umfasst, der eine Datenverknüpfung zwischen der Position des Elements und eines Referenzdatenmusters mit K Elementen umfasst, wobei ein Referenzdatenmuster durch Kombinieren des Elementwerts an der Position in der Referenzdatenbank und den Elementwerten von K-1 benachbarten Positionen des Elements in der Referenzdatenstruktur erhalten wird, wobei K ein ganze Zahl kleiner als oder gleich P+1 ist;
- Sortieren der Indexstruktur basierend auf den Referenzdatenmustern, die jeder der Datenverknüpfungen zugeordnet sind, um einen sortierten Index zu erhalten, wobei der sortierte Index einen Dateneintrag für jedes der N Referenzelemente der Referenzdatenstruktur umfasst, wobei ein Dateneintrag mindestens eine Positionsinformation umfasst, welche die Position eines Referenzelements in der Referenzdatenstruktur angibt; und

die Suchaktion umfasst:

- Lesen der Positionsinformation aus einem Eintrag der sortierten Verknüpfungsstruktur;
- Erzeugen eines Referenzdatenmusters von M Elementen aus der Referenzdatenstruktur unter Verwendung der Positionsinformation;
- Vergleichen des Datenmusters mit dem Referenzdatenmuster; und
- Anhalten der Suche, wenn im Index der Eintrag mit dem längsten Anfangsabschnitt des entsprechenden Referenzdatenmusters gefunden wird, der mit einem entsprechenden Anfangsabschnitt des Datenmusters übereinstimmt, oder kein übereinstimmender Anfangsabschnitt gefunden werden kann, und
- Bereitstellen der Position des Eintrags mit dem längsten Anfangsabschnitt eines entsprechenden Referenzdatenmusters, der mit einem entsprechenden Anfangsabschnitt des Datenmusters übereinstimmt, **dadurch gekennzeichnet, dass** das Verfahren ferner umfasst:

- Abrufen einer Sprungtabelle mit $2^J$ Einträgen, wobei J eine ganze Zahl ist und der Anzahl von Bits entspricht, die einen Anfangsabschnitt eines Referenzdatenmusters darstellen, wobei jeder Eintrag in der Sprungtabelle eine Zeilennummer, ein vorliegendes Feld und ein Sprungfeld aufweist, wobei das vorliegende Feld verwendet wird, um anzugeben, ob der sortierte Index einen Dateneintrag umfasst, der einem Referenzdatenmuster zugeordnet ist, dessen Anfangsabschnitt gleich den Bits ist, welche die Zeilennummer darstellen, wobei, wenn das vorliegende Feld angibt, dass der sortierte Index einen Dateneintrag umfasst, der einem Referenzdatenmuster zugeordnet ist, dessen Anfangsabschnitt gleich den Bits ist, welche die Zeilennummer darstellen, ein Sprungwert in dem Sprungfeld gespeichert wird, dessen Sprungwert sich auf den ersten Eintrag in dem sortierten Index bezieht, dessen jeweiliges Referenzdatenmuster einen Anfangsabschnitt mit einer vorbestimmten Größe aufweist, der mit den Bits übereinstimmt, die die Zeilennummer des Eintrags in der Sprungtabelle darstellen, und wobei die Suchaktion ferner umfasst:

- Abrufen eines Anfangsabschnitts mit der vorbestimmten Größe aus dem Datenmuster;
- Abrufen eines ersten Sprungwerts aus dem Eintrag der Sprungtabelle, dessen die Bits darstellende Zeilennummer dem Anfangsabschnitt des Datenmusters entspricht, und eines zweiten Sprungwerts, der dem nächsten Eintrag des Sprungwerts entspricht, dessen vorliegendes Feld angibt, dass der sortierte Index einen einem Referenzdatenmuster zugeordneten Dateneintrag umfasst, dessen Anfangsabschnitt äquivalent zu den Bits ist, welche die Zeilennummer darstellen;
- Verwenden des ersten und zweiten Sprungwerts, um einen Suchbereich in dem sortierten Index zu definieren.

8. Verfahren nach Anspruch 7, wobei ein Dateneintrag des sortierten Index ferner ein Prüffeld umfasst, dessen Wert den Elementwerten eines Abschnitts des Referenzdatenmusters entspricht, welcher der Positionsinformation des Dateneintrags zugeordnet ist; wobei die Suchaktion ferner das Lesen des Wertes des Prüffelds aus dem Eintrag des sortierten Index umfasst, und die Erzeugungsaktion ausführt, wenn ein vordefinierter Abschnitt des Datenmusters mit dem Wert des Prüffelds übereinstimmt.

9. Verfahren nach Anspruch 8, wobei der Wert des Prüffelds den Elementwerten eines Abschnitts des Referenzdatenmusters entspricht, welcher dem Anfangsabschnitt des Datenmusters folgt, das durch eine Zeilennummer der Sprungtabelle dargestellt wird.

10. Verfahren nach einem der Ansprüche 7 - 9, wobei ein Dateneintrag des sortierten Index ferner ein oder mehrere Zählfelder umfasst, wobei ein Wert eines Zählfelds die Anzahl von Einträgen in dem sortierten Index darstellt, deren zugeordnete Datenmuster von K Elementen identisch mit mindestens einem der Folgenden ist: das dem Eintrag zugeordnete Datenmuster, das dem Eintrag in umgekehrter komplementärer Reihenfolge zugeordnete Datenmuster, das dem Eintrag zugeordnete komplementäre Datenmuster, das umgekehrte Datenmuster des dem Eintrag zugeordneten Datenmusters; und wobei das Verfahren ferner umfasst:

- Auslesen des einen oder der mehreren Zählfelder aus dem Eintrag, dessen zugeordnetes Referenzdatenmuster mit dem Datenmuster übereinstimmt, und Speichern einer entsprechenden Verknüpfung zwischen einer Darstellung des Datenmusters und dem entsprechenden Wert des einen oder der mehreren Zählfelder in einem Datenspeicher zur weiteren Verarbeitung.

11. Verfahren nach einem der Ansprüche 7 - 10, wobei die Referenzdatenstruktur und Indexstruktur aus einem Datenspeicher abgerufen werden, in dem eine durch das Verfahren nach einem der Ansprüche 1 - 6 erhaltene Referenzindexdatenstruktur erfasst ist.

12. Computerimplementiertes System (400), umfassend einen Prozessor (410), ein Eingabe-/Ausgabegerät (430), eine Datenbank (440) und einen mit dem Prozessor verbundenen Datenspeicher (420), wobei der Datenspeicher Befehle umfasst, die bei Ausführung durch den Prozessor (410) das computerimplementierte System veranlassen, die Verfahren nach einem der Ansprüche 1 - 11 durchzuführen.

13. Computerprogrammprodukt, umfassend Anweisungen, die durch eine Computeranordnung geladen werden können, welche die Computeranordnung veranlassen, eines der Verfahren gemäß den Ansprüchen 1 - 11 durchzuführen.

14. Mit einem Computerprogrammprodukt bereitgestelltes prozessorlesbares Medium, umfassend Anweisungen, die durch eine Computeranordnung geladen werden können, welche die Computeranordnung veranlassen, eines der Verfahren gemäß den Ansprüchen 1 - 11 durchzuführen.

15. Datenbankprodukt, umfassend eine Referenzindexdatenstruktur, die durch eines der Verfahren gemäß den Ansprüchen 1 - 6 erzeugt wurde.

**Revendications**

1. Procédé mis en oeuvre par ordinateur de génération d'une structure de données d'index de référence permettant de trouver des positions associées de M éléments d'un motif de données dans une structure de données de référence, la structure de données de référence comprend N éléments de référence et P éléments de remplissage après le dernier élément de référence, chaque élément ayant une position dans la structure de données de référence et comprend une valeur d'élément associé et dans lequel N >> M, le procédé consistant :

- à récupérer la structure de données de référence ;
- à générer, à partir de la structure de données de référence pour chacun des N éléments de référence dans la structure de données de référence, une structure d'index qui comprend pour chacun des N éléments de référence une entrée d'index qui définit une liaison de données entre une position d'un élément dans la structure de données de référence et un motif de données de référence, le motif de données de référence comprend K éléments qui sont obtenus en combinant la valeur d'élément à ladite position dans la base de données de référence et les valeurs d'élément de K - 1 positions voisines de l'élément dans la structure de données de référence, K étant un nombre entier inférieur ou égal à P + 1 ;
- à trier la structure d'index sur la base des motifs de données de référence associés à chacune des liaisons de données pour obtenir un index trié, l'index trié comprend une entrée de données pour chacun des N éléments de référence de la structure de données de référence, une entrée de données comprend au moins des informations de position indiquant la position d'un élément de référence dans la structure de données de référence ; et
- à trier l'index trié et la structure de données de référence dans un stockage de données en tant que structure de données d'index de référence,

**caractérisé en ce que** le procédé consiste en outre :

- à générer une table de sauts avec $2^J$ entrées, J est un nombre entier et correspond au nombre de bits représentant une partie initiale d'un motif de données de référence, chaque entrée de la table de sauts ayant un nombre de rangées, un champ actuel et un champ saut, le champ actuel est utilisé pour indiquer si l'index trié comprend une entrée de données associée à un motif de données de référence dont une partie initiale est égale aux bits représentant le nombre de rangées, si le champ actuel indique que l'index trié comprend une entrée de données associée à un motif de données de référence dont une partie initiale est égale aux bits représentant le nombre de rangées, une valeur de saut est stockée dans le champ saut dont une valeur de saut se réfère à la première entrée dans l'index trié dont un motif de données de référence correspondant présente une partie initiale qui correspond aux bits représentant le nombre de rangées de ladite entrée de la table de sauts et
- à ajouter la table de sauts à la structure de données d'index de référence.

2. Procédé selon la revendication 1, dans lequel une entrée de données de l'index trié comprend en outre un champ sonde dont une valeur correspond aux valeurs d'élément d'une partie du motif de données de référence associé aux informations de position de ladite entrée de données.

3. Procédé selon la revendication 2, dans lequel la valeur du champ sonde correspond aux valeurs d'élément d'une partie du motif de données de référence qui est ultérieure à la partie initiale du motif de données représenté par un nombre de rangées de la table de sauts.

4. Procédé selon l'une quelconque des revendications 1 - 3, dans lequel une entrée de données de l'index trié comprend en outre un ou plusieurs champs de comptage, le procédé consiste en outre :

- à déterminer pour chaque entrée dans l'index trié le nombre d'entrées dans l'index trié dont un motif de données associé est identique au motif de données associé à ladite entrée ;
- à déterminer pour chaque entrée dans l'index trié le nombre d'entrées dans l'index trié dont un motif de données associé est identique au motif de données associé à ladite entrée dans un ordre de complément inverse ;
- à déterminer pour chaque entrée dans l'index trié le nombre d'entrées dans l'index trié dont un motif de données associé est identique au complément et/ou au motif de données inverse du motif de données associé à ladite entrée ; et
- à stocker un ou plusieurs des nombres déterminés et/ou une somme d'un ou de plusieurs des nombres déterminés dans le ou les champs de comptage.

5. Procédé selon l'une quelconque des revendications 1 - 4, dans lequel la récupération de l'action de la structure de données de référence consiste :

- à lire un nombre de séquences de référence à partir d'un ou de plusieurs fichiers de données de référence ;
- à générer la structure de données de référence en ajoutant alternativement une séquence de référence et un motif prédéfini de P éléments de remplissage à la structure de données de référence.

6. Procédé selon l'une quelconque des revendications 1 - 5, dans lequel la structure de données d'index de référence est sous la forme d'un fichier de données.

7. Procédé mis en oeuvre par ordinateur pour trouver des positions associées de M éléments d'un motif de données dans une structure de données de référence, qui est dérivée d'un ou plusieurs fichiers de données de référence ; le procédé consiste :

- à récupérer une structure d'index qui est dérivée de la structure de données de référence ;
- à faire des recherches dans la structure d'index pour trouver la position des M éléments du motif de données dans la structure de données de référence ; et
- à stocker le motif de données et des informations de position trouvées pour un traitement supplémentaire, la structure de données de référence comprend N éléments de référence et P éléments de remplissage après le dernier élément de référence, chaque élément ayant une position dans la structure de données de référence et comprend une valeur d'élément associé et dans lequel N >> M,

la structure d'index est un index trié qui a été généré par :

- la génération, à partir de la structure de données de référence pour chacun des N éléments de référence dans la structure de données de référence, une structure d'index qui comprend pour chacun des N éléments de référence une entrée d'index qui définit une liaison de données entre la position de l'élément et un motif de données de référence comprenant K éléments, un motif de données de référence est obtenu en combinant la valeur d'élément à ladite position dans la base de données de référence et les valeurs d'élément de K - 1 positions voisines de l'élément dans la structure de données de référence, K étant un nombre entier inférieur ou égal à P + 1 ;
- à trier la structure d'index sur la base des motifs de données de référence associés à chacune des liaisons de données pour obtenir un index trié, l'index trié comprend une entrée de données pour chacun des N éléments de référence de la structure de données de référence, une entrée de données comprend au moins des informations de position indiquant la position d'un élément de référence dans la structure de données de référence ; et

l'action de recherche consiste :

- à lire des informations de position à partir d'une entrée de la structure de liaison triée ;
- à générer un motif de données de référence de M éléments à partir de la structure de données de référence à l'aide des informations de position ;
- à comparer le motif de données avec le motif de données de référence ; et
- à arrêter la recherche lorsque l'entrée dans l'index est trouvée avec la partie initiale la plus longue d'un motif de données de référence correspondant qui correspond à une partie initiale correspondante du motif de données ou qu'aucune partie initiale mise en correspondance ne peut être trouvée et
- à fournir la position de l'entrée avec la partie initiale la plus longue d'un motif de données de référence correspondant qui correspond à une partie initiale correspondante du motif de données,

**caractérisé en ce que** le procédé consiste en outre :

- à récupérer une table de sauts avec $2^J$ entrées, J est un nombre entier et correspond au nombre de bits représentant une partie initiale d'un motif de données de référence, chaque entrée de la table de sauts ayant un nombre de rangées, un champ actuel et un champ saut, le champ actuel est utilisé pour indiquer si l'index trié comprend une entrée de données associée à un motif de données de référence dont une partie initiale est égale aux bits représentant le nombre de rangées, si le champ actuel indique que l'index trié comprend une entrée de données associée à un motif de données de référence dont une partie initiale est égale aux bits représentant le nombre de rangées, une valeur de saut est stockée dans le champ saut dont une valeur de saut se réfère à la première entrée dans l'index trié dont un motif de données de référence correspondant présente une partie initiale avec une taille prédéterminée qui correspond aux bits représentant le nombre de rangées de ladite entrée de la table de sauts, et l'action de recherche consiste en outre :
- à extraire une partie initiale avec la taille prédéterminée du motif de données ;
- à extraire une première valeur de saut de l'entrée de la table de sauts dont des bits représentant le nombre de rangées correspondent à la partie initiale du motif de données et une deuxième valeur de saut correspondant à l'entrée suivante de la valeur de saut, dont le champ actuel indique que l'index trié comprend une entrée de données associée à un motif de données de référence, laquelle partie initiale est équivalente aux bits représentant le nombre de rangées ;
- à utiliser la première et la seconde valeur de saut pour définir une zone de recherche dans l'index trié.

8. Procédé selon la revendication 7, dans lequel une entrée de données de l'index trié comprend en outre un champ sonde dont une valeur correspond aux valeurs d'élément d'une partie du motif de données de référence associé aux informations de position de ladite entrée de données ; l'action de recherche consiste en outre à lire la valeur du champ sonde à partir de l'entrée de l'index trié ; et effectue l'action de génération si une partie prédéfinie du motif de données correspond à la valeur du champ sonde.

9. Procédé selon la revendication 8, dans lequel la valeur du champ sonde correspond aux valeurs d'élément d'une partie du motif de données de référence qui est ultérieure à la partie initiale du motif de données qui est représenté par un nombre de rangées de la table de sauts.

10. Procédé selon l'une quelconque des revendications 7 - 9, dans lequel une entrée de données de l'index trié comprend en outre un ou plusieurs champs de comptage, une valeur d'un champ de comptage représente le nombre d'entrées dans l'index trié dont un motif de données associé de K éléments est identique : au motif de données associé à ladite entrée et/ou au motif de données associé à ladite entrée dans un ordre de complément inverse et/ou au motif de données de complément associé à ladite entrée et/ou au motif de données inverse du motif de données associé à ladite entrée ; et dans lequel le procédé consiste en outre à :

- lire le ou les champs de comptage à partir de l'entrée dont un motif de données de référence associé correspond au motif de données et stocker une liaison correspondante entre une représentation du motif de données et la valeur correspondante du ou des champs de comptage dans un stockage de données pour un traitement supplémentaire.

11. Procédé selon l'une quelconque des revendications 7 - 10, dans lequel la structure de données de référence et la structure d'index sont extraites d'un stockage de données dans lequel une structure de données d'index de référence obtenue par le procédé selon l'une quelconque des revendications 1 - 6 est enregistrée.

12. Système mis en oeuvre par ordinateur (400) comprenant un processeur (410), un dispositif d'entrée/de sortie (430), une base de données (440) et un stockage de données (420) raccordé au processeur, le stockage de données comprenant des instructions qui, lorsqu'elles sont exécutées par le processeur (410), contraignent le système mis en oeuvre par ordinateur à réaliser les procédés selon l'une quelconque des revendications 1 - 11.

13. Produit-programme d'ordinateur comprenant des instructions qui peuvent être chargées par un agencement d'ordinateur, contraignant ledit agencement d'ordinateur à réaliser l'un quelconque des procédés selon l'une quelconque des revendications 1 - 11.

14. Support lisible par un processeur pourvu d'un produit-programme d'ordinateur comprenant des instructions qui peuvent être chargées par un agencement d'ordinateur, contraignant ledit agencement d'ordinateur à réaliser l'un quelconque des procédés selon les revendications 1 - 11.

15. Produit de base de données comprenant une structure de données d'index de référence générée par l'un quelconque des procédés selon les revendications 1 - 6.

| pos | ref_pat | forw | rev-comp | rev | forw-comp | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | 36 | 37 | 38 | 39 | 40 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | | | | | | T | C | A | G | A | G | T | C | G | A | G | A | G | A | C | C | A | A | A | A | G | C | T | G | A | G | T | G | A | C | C | T | C | A | G | T | A | A | A | A |
| 1 | TCAGA | TCAG | CTGA | GACT | AGTC | T | C | A | G | A | | | | | | | | | | | | | | | | | | | | | | | | | | | | | | | | | | | | |
| 2 | CAGAG | CAGA | TCTG | AGAC | GTCT | | C | A | G | A | G | | | | | | | | | | | | | | | | | | | | | | | | | | | | | | | | | | |
| 3 | AGAGT | AGAG | CTCT | GAGA | TCTC | | | A | G | A | G | T | | | | | | | | | | | | | | | | | | | | | | | | | | | | | | | | | |
| 4 | GAGTC | GAGT | ACTC | TGAG | CTCA | | | | G | A | G | T | C | | | | | | | | | | | | | | | | | | | | | | | | | | | | | | | | |
| 5 | AGTCG | AGTC | GACT | CTGA | TCAG | | | | | A | G | T | C | G | | | | | | | | | | | | | | | | | | | | | | | | | | | | | | |
| 6 | GTCGA | GTCG | CGAC | GCTG | CAGC | | | | | | G | T | C | G | A | | | | | | | | | | | | | | | | | | | | | | | | | | | | | |
| 7 | TCGAG | TCGA | TCGA | AGCT | AGCT | | | | | | | T | C | G | A | G | | | | | | | | | | | | | | | | | | | | | | | | | | | | |
| 8 | CGAGA | CGAG | CTCG | GAGC | GCTC | | | | | | | | C | G | A | G | A | | | | | | | | | | | | | | | | | | | | | | | | | | | |
| 9 | GAGAG | GAGA | TCTC | AGAG | CTCT | | | | | | | | | G | A | G | A | G | | | | | | | | | | | | | | | | | | | | | | | | | | |
| 10 | AGAGA | AGAG | CTCT | GAGA | TCTC | | | | | | | | | | A | G | A | G | A | | | | | | | | | | | | | | | | | | | | | | | | | |
| 11 | GAGAC | GAGA | TCTC | AGAG | CTCT | | | | | | | | | | | G | A | G | A | C | | | | | | | | | | | | | | | | | | | | | | | |
| 12 | AGACC | AGAC | GTCT | CAGA | TCTG | | | | | | | | | | | | A | G | A | C | C | | | | | | | | | | | | | | | | | | | | | | |
| 13 | GACCA | GACC | GGTC | CCAG | CTGG | | | | | | | | | | | | | G | A | C | C | A | | | | | | | | | | | | | | | | | | | | | |
| 14 | ACCAA | ACCA | TGGT | ACCA | TGGT | | | | | | | | | | | | | | A | C | C | A | A | | | | | | | | | | | | | | | | | | | | |
| 15 | CCAAA | CCAA | TTGG | AACC | GGTT | | | | | | | | | | | | | | | C | C | A | A | A | | | | | | | | | | | | | | | | | | |
| 16 | CAAAA | CAAA | TTTG | AAAC | GTTT | | | | | | | | | | | | | | | | C | A | A | A | A | | | | | | | | | | | | | | | | | |
| 17 | AAAAG | AAAA | TTTT | AAAA | TTTT | | | | | | | | | | | | | | | | | A | A | A | A | G | | | | | | | | | | | | | | | | |
| 18 | AAAGC | AAAG | CTTT | GAAA | TTTC | | | | | | | | | | | | | | | | | | A | A | A | G | C | | | | | | | | | | | | | | | |
| 19 | AAGCT | AAGC | GCTT | CGAA | TTCG | | | | | | | | | | | | | | | | | | | A | A | G | C | T | | | | | | | | | | | | | | |
| 20 | AGCTG | AGCT | AGCT | TCGA | TCGA | | | | | | | | | | | | | | | | | | | | A | G | C | T | G | | | | | | | | | | | | | |
| 21 | GCTGA | GCTG | CAGC | GTCG | CGAC | | | | | | | | | | | | | | | | | | | | | G | C | T | G | A | | | | | | | | | | | | |
| 22 | CTGAG | CTGA | TCAG | AGTC | GACT | | | | | | | | | | | | | | | | | | | | | | C | T | G | A | G | | | | | | | | | | | |
| 23 | TGAGT | TGAG | CTCA | GAGT | ACTC | | | | | | | | | | | | | | | | | | | | | | | T | G | A | G | T | | | | | | | | | | |
| 24 | GAGTG | GAGT | ACTC | TGAG | CTCA | | | | | | | | | | | | | | | | | | | | | | | | G | A | G | T | G | | | | | | | | | |
| 25 | AGTGA | AGTG | CACT | GTGA | TCAC | | | | | | | | | | | | | | | | | | | | | | | | | A | G | T | G | A | | | | | | | | |
| 26 | GTGAC | GTGA | TCAC | AGTG | CACT | | | | | | | | | | | | | | | | | | | | | | | | | | G | T | G | A | C | | | | | | | |
| 27 | TGACC | TGAC | GTCA | CAGT | ACTG | | | | | | | | | | | | | | | | | | | | | | | | | | | T | G | A | C | C | | | | | | |
| 28 | GACCT | GACC | GGTC | CCAG | CTGG | | | | | | | | | | | | | | | | | | | | | | | | | | | | G | A | C | C | T | | | | | |
| 29 | ACCTC | ACCT | AGGT | TCCA | TGGA | | | | | | | | | | | | | | | | | | | | | | | | | | | | | A | C | C | T | C | | | | |
| 30 | CCTCA | CCTC | GAGG | CTCC | GGAG | | | | | | | | | | | | | | | | | | | | | | | | | | | | | | C | C | T | C | A | | | |
| 31 | CTCAG | CTCA | TGAG | ACTC | GAGT | | | | | | | | | | | | | | | | | | | | | | | | | | | | | | | C | T | C | A | G | | | |
| 32 | TCAGT | TCAG | CTGA | GACT | AGTC | | | | | | | | | | | | | | | | | | | | | | | | | | | | | | | | T | C | A | G | T | | |
| 33 | CAGTA | CAGT | ACTG | TGAC | GTCA | | | | | | | | | | | | | | | | | | | | | | | | | | | | | | | | | C | A | G | T | A | |
| 34 | AGTAA | AGTA | TACT | ATGA | TCAT | | | | | | | | | | | | | | | | | | | | | | | | | | | | | | | | | | A | G | T | A | A |
| 35 | GTAAA | GTAA | TTAC | AATG | CATT | | | | | | | | | | | | | | | | | | | | | | | | | | | | | | | | | | | G | T | A | A | A |
| 36 | TAAAA | TAAA | TTTA | AAAT | ATTT | | | | | | | | | | | | | | | | | | | | | | | | | | | | | | | | | | | | T | A | A | A | A |

FIG. 1

| Entry_nr | ref_pat | POS | probe | c1 | c2 | c3 | c4 |
|---|---|---|---|---|---|---|---|
| 1 | AAAAG | 17 | A | 1 | 0 | 1 | 0 |
| 2 | AAAGC | 18 | A | 1 | 0 | 0 | 0 |
| 3 | AAGCT | 19 | G | 1 | 0 | 0 | 0 |
| 4 | ACCAA | 14 | C | 1 | 0 | 1 | 0 |
| 5 | ACCTC | 29 | C | 1 | 0 | 0 | 0 |
| 6 | AGACC | 12 | A | 1 | 0 | 1 | 0 |
| 7 | AGAGA | 10 | A | 2 | 0 | 2 | 0 |
| 8 | AGAGT | 3 | A | 2 | 0 | 2 | 0 |
| 9 | AGCTG | 20 | C | 1 | 1 | 1 | 1 |
| 10 | AGTAA | 34 | T | 1 | 0 | 0 | 0 |
| 11 | AGTCG | 5 | T | 1 | 0 | 1 | 2 |
| 12 | AGTGA | 25 | T | 1 | 0 | 1 | 0 |
| 13 | CAAAA | 16 | A | 1 | 0 | 0 | 0 |
| 14 | CAGAG | 2 | G | 1 | 0 | 1 | 0 |
| 15 | CAGTA | 33 | G | 1 | 0 | 1 | 0 |
| 16 | CCAAA | 15 | A | 1 | 0 | 0 | 0 |
| 17 | CCTCA | 30 | T | 1 | 0 | 0 | 0 |
| 18 | CGAGA | 8 | A | 1 | 0 | 0 | 0 |
| 19 | CTCAG | 31 | C | 1 | 1 | 0 | 2 |
| 20 | CTGAG | 22 | G | 1 | 2 | 1 | 0 |
| 21 | GACCA | 13 | C | 2 | 0 | 0 | 0 |
| 22 | GACCT | 28 | C | 2 | 0 | 0 | 0 |
| 23 | GAGAC | 11 | G | 2 | 0 | 2 | 0 |
| 24 | GAGAG | 9 | G | 2 | 0 | 2 | 0 |
| 25 | GAGTC | 4 | G | 2 | 0 | 1 | 1 |
| 26 | GAGTG | 24 | G | 2 | 0 | 1 | 1 |
| 27 | GCTGA | 21 | T | 1 | 0 | 1 | 0 |
| 28 | GTAAA | 35 | A | 1 | 0 | 0 | 0 |
| 29 | GTCGA | 6 | C | 1 | 0 | 1 | 0 |
| 30 | GTGAC | 26 | G | 1 | 0 | 1 | 0 |
| 31 | TAAAA | 36 | A | 1 | 0 | 0 | 0 |
| 32 | TCAGA | 1 | A | 2 | 1 | 0 | 1 |
| 33 | TCAGT | 32 | A | 2 | 1 | 0 | 1 |
| 34 | TCGAG | 7 | G | 1 | 1 | 1 | 1 |
| 35 | TGACC | 27 | A | 1 | 0 | 1 | 0 |
| 36 | TGAGT | 23 | A | 1 | 1 | 2 | 0 |

FIG. 2

| Entry | Row_val | present | JMP-Val |
|-------|---------|---------|---------|
| 1 | AA | 1 | 1 |
| 2 | AC | 1 | 4 |
| 3 | AG | 1 | 6 |
| 4 | AT | 0 | 13 |
| 5 | CA | 1 | 13 |
| 6 | CC | 1 | 16 |
| 7 | CG | 1 | 18 |
| 8 | CT | 1 | 19 |
| 9 | GA | 1 | 21 |
| 10 | GC | 1 | 27 |
| 11 | GG | 0 | 28 |
| 12 | GT | 1 | 28 |
| 13 | TA | 1 | 31 |
| 14 | TC | 1 | 32 |
| 15 | TG | 1 | 35 |
| 16 | TT | 0 | 36 |

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010104608 A2

**Non-patent literature cited in the description**

- **NING et al.** *Genome,* 2001, vol. 11, 1725-1729